# EUROPEAN PATENT APPLICATION

(11) **EP 2 428 220 A1**
(43) Date of publication of application: **14.03.2012**
(21) Application number: 11180973.7
(22) Date of filing: 12.09.2011
(51) Int. Cl.: A61K 39/12, C07K 14/18, A61P 31/14

(54) **Dengue vaccine, pharmaceutical composition comprising the same, and nucleotide molecule**

(30) Priority: 13.09.2010 TW 099130861
(71) Applicant: National Cheng Kung University, Tainan City 701 (TW)
(72) Inventor: Lin, Yee-Shin, 701 Tainan City (TW)
(74) Representative: Leathley, Anna Elisabeth

(57) **Abstract**

The present invention relates to a dengue vaccine, a pharmaceutical composition including the same, and a nucleotide molecule. The dengue vaccine includes N and C termini-deleted nonstructural protein ΔNC NS1 with a peptide fragment from amino acids 36 to 270, which is derived from dengue virus nonstructural protein 1 (DV NS1) with deletions of N-terminal region from amino acids 1 to 35 and C-terminal region from amino acids 271 to 352. The dengue vaccine of the present invention is depleted of cross-reactivity with endothelial cells and platelets, and can shorten the bleeding time.

<110>  National Cheng Kung University

<120>  DENGUE VACCINE, PHARMACEUTICAL COMPOSITION COMPRISING THE SAME, AND
NUCLEOTIDE MOLECULE

<130>  1.13.110522

<150>  TW 099130861
<151>  2010-09-13

<160>  4

<170>  Patent In version 3.3

<210>  1
<211>  235
<212>  PRT
<213>  Dengue virus

<400>  1

Pro Glu Ser Pro Ser Lys Leu Ala Ser Ala Ile Gln Lys Ala His Glu
1               5                   10                  15

Glu Gly Ile Cys Gly Ile Arg Ser Val Thr Arg Leu Glu Asn Leu Met
            20                  25                  30

Trp Lys Gln Ile Thr Pro Glu Leu Asn His Ile Leu Ser Glu Asn Glu
        35                  40                  45

Val Lys Leu Thr Ile Met Thr Gly Asp Ile Lys Gly Ile Met Gln Ala
    50                  55                  60

Gly Lys Arg Ser Leu Arg Pro Gln Pro Thr Glu Leu Lys Tyr Ser Trp
65                  70                  75                  80

Lys Thr Trp Gly Lys Ala Lys Met Leu Pro Thr Glu Ser His Asn Gln
                85                  90                  95

Thr Phe Leu Ile Asp Gly Pro Glu Thr Ala Glu Cys Pro Asn Thr Asn
            100                 105                 110

Arg Ala Trp Asn Ser Leu Glu Val Glu Asp Tyr Gly Phe Gly Val Phe
        115                 120                 125

Thr Thr Asn Ile Trp Leu Lys Leu Arg Glu Lys Gln Asp Val Phe Cys
    130                 135                 140

Asp Ser Lys Leu Met Ser Ala Ala Ile Lys Asp Asn Arg Ala Val His
145                 150                 155                 160

Ala Asp Met Gly Tyr Trp Ile Glu Ser Ala Leu Asn Asp Thr Trp Lys
                165                 170                 175

Ile Glu Lys Ala Ser Phe Ile Glu Val Lys Ser Cys His Trp Pro Lys
            180                 185                 190

Pro His Thr Leu Trp Ser Asn Gly Val Leu Glu Ser Glu Met Ile Ile
        195                 200                 205

Pro Lys Asn Phe Ala Gly Pro Val Ser Gln His Asn Tyr Arg Pro Gly
    210                 215                 220

Tyr His Thr Gln Thr Ala Gly Pro Trp His Leu
225                 230                 235

<210>  2
<211>  705
<212>  DNA
<213>  Dengue virus

<400>  2
ccagaatccc cttcaaaact agcttcagct atccagaaag ctcatgaaga gggcatttgt      60

ggaatccgct cagtaacaag actggaaaat ctgatgtgga aacaaataac accagaattg     120

aatcacattc tatcagaaaa tgaggtgaag ttgactatta tgacaggaga catcaaagga     180

atcatgcagg caggaaaacg atctctgcgg ccccagccca ctgagctgaa gtattcatgg     240

aaaacatggg gcaaagcgaa aatgctccct acagagtctc ataaccagac ctttctcatt     300

gatggccccg aaacagcaga atgccccaac acaaacagag cttggaattc gctggaagtt     360

gaagactatg gctttggagt attcaccacc aatatatggc taaagttgag agaaaagcag     420

gatgtattct gcgactcaaa actcatgtca gcggccataa aagacaacag agccgtccat     480

gccgatatgg gttattggat agaaagtgca ctcaatgaca catggaagat agagaaagcc     540

tctttcatcg aagttaaaag ctgccactgg ccaaagccac acaccctctg gagtaatgga     600

gtgctagaaa gtgagatgat aattccaaag aatttcgctg gaccagtgtc acaacacaac     660

tacagaccag gctaccatac acaaacagca ggaccatggc atcta                     705

<210>  3
<211>  352
<212>  PRT
<213>  Dengue virus

<400>  3

Asp Ser Gly Cys Val Val Ser Trp Lys Asn Lys Glu Leu Lys Cys Gly
1               5                   10                  15

Ser Gly Ile Phe Ile Thr Asp Asn Val His Thr Trp Thr Glu Gln Tyr
            20                  25                  30

Lys Phe Gln Pro Glu Ser Pro Ser Lys Leu Ala Ser Ala Ile Gln Lys
        35                  40                  45

Ala His Glu Glu Gly Ile Cys Gly Ile Arg Ser Val Thr Arg Leu Glu
    50                  55                  60

Asn Leu Met Trp Lys Gln Ile Thr Pro Glu Leu Asn His Ile Leu Ser

65                  70                  75                  80

Glu Asn Glu Val Lys Leu Thr Ile Met Thr Gly Asp Ile Lys Gly Ile
                85                  90                  95

Met Gln Ala Gly Lys Arg Ser Leu Arg Pro Gln Pro Thr Glu Leu Lys
            100                 105                 110

Tyr Ser Trp Lys Thr Trp Gly Lys Ala Lys Met Leu Pro Thr Glu Ser
        115                 120                 125

His Asn Gln Thr Phe Leu Ile Asp Gly Pro Glu Thr Ala Glu Cys Pro
    130                 135                 140

Asn Thr Asn Arg Ala Trp Asn Ser Leu Glu Val Glu Asp Tyr Gly Phe
145                 150                 155                 160

Gly Val Phe Thr Thr Asn Ile Trp Leu Lys Leu Arg Glu Lys Gln Asp
                165                 170                 175

Val Phe Cys Asp Ser Lys Leu Met Ser Ala Ala Ile Lys Asp Asn Arg
            180                 185                 190

Ala Val His Ala Asp Met Gly Tyr Trp Ile Glu Ser Ala Leu Asn Asp
        195                 200                 205

Thr Trp Lys Ile Glu Lys Ala Ser Phe Ile Glu Val Lys Ser Cys His
    210                 215                 220

Trp Pro Lys Pro His Thr Leu Trp Ser Asn Gly Val Leu Glu Ser Glu
225                 230                 235                 240

Met Ile Ile Pro Lys Asn Phe Ala Gly Pro Val Ser Gln His Asn Tyr
                245                 250                 255

Arg Pro Gly Tyr His Thr Gln Thr Ala Gly Pro Trp His Leu Gly Lys
            260                 265                 270

Leu Glu Met Asp Phe Asp Phe Cys Glu Gly Thr Thr Val Val Val Thr
        275                 280                 285

Glu Asp Cys Gly Asp Arg Gly Pro Ser Leu Arg Thr Thr Thr Ala Ser
    290                 295                 300

Gly Lys Leu Ile Thr Glu Trp Cys Cys Arg Ser Cys Thr Leu Pro Pro
305                 310                 315                 320

Leu Arg Tyr Arg Gly Glu Asp Gly Cys Trp Tyr Gly Met Glu Ile Arg
                325                 330                 335

Pro Leu Lys Glu Lys Glu Glu Asn Leu Val Asn Ser Leu Val Thr Ala

340                 345                 350

<210>  4
<211>  1056
<212>  DNA
<213>  Dengue virus

<400>  4
gatagtggtt gcgttgtgag ctggaaaaac aaagaactga agtgtggcag tgggattttc      60

atcacagaca acgtgcacac atggacagaa caatacaagt tccaaccaga atccccttca     120

aaactagctt cagctatcca gaaagctcat gaagagggca tttgtggaat ccgctcagta     180

acaagactgg aaaatctgat gtggaaacaa ataacaccag aattgaatca cattctatca     240

gaaaatgagg tgaagttgac tattatgaca ggagacatca aaggaatcat gcaggcagga     300

aaacgatctc tgcggcccca gcccactgag ctgaagtatt catggaaaac atggggcaaa     360

gcgaaaatgc tccctacaga gtctcataac cagacctttc tcattgatgg ccccgaaaca     420

gcagaatgcc ccaacacaaa cagagcttgg aattcgctgg aagttgaaga ctatggcttt     480

ggagtattca ccaccaatat atggctaaag ttgagagaaa agcaggatgt attctgcgac     540

tcaaaactca tgtcagcggc cataaaagac aacagagccg tccatgccga tatgggttat     600

tggatagaaa gtgcactcaa tgacacatgg aagatagaga aagcctcttt catcgaagtt     660

aaaagctgcc actggccaaa gccacacacc ctctggagta atggagtgct agaaagtgag     720

atgataattc caaagaattt cgctggacca gtgtcacaac acaactacag accaggctac     780

catacacaaa cagcaggacc atggcatcta ggtaagcttg agatggactt tgatttctgc     840

gaaggaacca cagtggtggt gactgaggac tgtggagata gaggaccctc tttaagaaca     900

actactgcct ctggaaaact cataacagaa tggtgctgcc gatcttgcac attaccaccg     960

ctaagataca gaggtgagga cggatgctgg tacgggatgg aaatcagacc attgaaagag    1020

aaagaagaga atttggtcaa ctccttggtc acagcc                              1056

## Description

### CROSS REFERENCE TO RELATED APPLICATION

This application claims the benefits of the Taiwan Patent Application Serial Number 099130861, filed on September 13,2010, the subject matter of which is incorporated herein by reference.

### BACKGROUND OF THE INVENTION

### 1. Field of the Invention

The present invention relates to a dengue vaccine, a pharmaceutical composition comprising the same and a nucleotide sequence, more particularly, to a dengue vaccine with reduced cross-reactivity with endothelial cells and platelets, a pharmaceutical composition comprising the same, and a nucleotide molecule.

### 2. Description of Related Art

Dengue fever, also called breakbone fever, is an acute infectious disease induced by the propagation of dengue viruses (DV) via *Aedes aegypti* or *Aedes albopictus* and its symptoms include high fever (39 °C to 40 °C) or aversion to cold, skin rash, fatigue in limbs, muscle pain, frontal headache, retro-orbital pain, abdominal pain, backache (hence the term breakbone fever), sore throat, and maybe vomiting and fainting. The commonly mentioned dengue virus is classic dengue fever, also called primary dengue fever. In addition, severe and life-threatening dengue fever characterized by hemorrhage or shock may be developed, also called dengue hemorrhagic fever (DHF) or dengue shock syndrome (DSS), or secondary dengue.

It is estimated that there are about 50 million to 100 million cases of dengue infection worldwide each year, with about 250,000 to 500,000 cases of dengue hemorrhagic fever. In other words, two-fifths of the world's population is at risk from dengue infection. Hence, the prevention and treatment of dengue fever is an important issue for the governments of many countries. However, the mechanism underlying dengue disease is not clearly understood. Furthermore, neither effective and safe vaccines nor drugs on specific treatment of diseases caused by dengue viruses are available so far. Since dengue virus is the major pathogen of dengue disease, the early detection or prevention with effective vaccine can efficiently control morbidity and death rates of dengue fever.

Recently, numerous strategies of dengue vaccine design are based on the neutralizing efficacy of antibodies against viral envelope (E) or nonstructure (NS) protein. Although the E protein is responsible for eliciting major neutralizing antibodies during DV infection, it is also associated with the induction of antibody dependent enhancement (ADE). Antibody dependent enhancement (ADE) is a complicating factor in dengue vaccine development in that the pre-existing antibodies raise concerns of causing more severe disease. In contrast, antibodies against the NS 1 protein are able to kill DV-infected cells by complement-induced cell lysis and thus are not associated with ADE. A limitation to the vaccine strategy of NS1, however, is that anti-NS1 antibodies may cause cross-reaction with endothelial cells and platelets, and thus may negatively influence coagulation function, resulting in prolonged bleeding time. The side effect of autoimmunity caused by the vaccine still has not been resolved.

Therefore, it is desirable for the technical field to develop a novel vaccine which does not induce antibody dependent enhancement and autoimmunity, is depleted of cross-reactivity with endothelial cells and platelets, and is able to shorten bleeding time.

### SUMMARY OF THE INVENTION

The dengue vaccine according to the present invention includes C and N termini-deleted nonstructural protein Δ NC NS1 with a peptide fragment from amino acids 36 to 270, which is derived from dengue virus nonstructural protein 1 (DVNS1) with deletions of N-terminal region from amino acids 1 to 35 and C-terminal region from amino acids 271 to 352.

Through sequence alignment analysis, the inventors of the present invention found that the N-terminal and C-terminal regions of DV NS 1 protein contain epitopes involved in cross-reactivity to target proteins. Therefore, the inventors of the present invention used N terminus (aa 1-35)-deleted and C terminus (aa 271-352)-deleted DV ΔNC NS1 protein as a vaccine to reduce the cross-reactivity with endothelial cells and platelets and thus to shorten bleeding time. In addition, DV ΔNC NS1 protein of the present invention is derived from dengue virus nonstructural protein rather than dengue virus envelope protein, and thus can be used as an inventive and practical vaccine without antibody dependent enhancement and autoimmunity.

In the dengue vaccine of the present invention, the N and C termini-deleted nonstructural protein ΔNC NS1 preferably has the amino acid sequence of SEQ.ID.NO.1.

In the dengue vaccine of the present invention, the N and C termini-deleted nonstructural protein Δ NC NS1 preferably is derived from dengue virus protein. The dengue virus protein is, for example, SEQ.ID.NO.3 in the sequence listing.

In the dengue vaccine of the present invention, the dengue vaccine preferably is used to prevent dengue hemorrhagic fever or dengue shock syndrome.

In the dengue vaccine according to the present invention, the amino acid sequence similarity or identity between the N and C termini-deleted nonstructural protein ΔNC NS1 and the SEQ.ID.NO.1 preferably is 90% or more, and more preferably is 95% or more.

The present invention further provides a dengue vaccine-containing pharmaceutical composition. The dengue vaccine includes N and C termini-deleted nonstructural protein ΔNC NS1 with a peptide fragment from amino acids 36 to 270, which is derived from dengue virus nonstructural protein 1 (DVNS1) with deletions of N-terminal region from amino acids 1 to 35 and C-terminal region from amino acids 271 to 352.

The dengue vaccine-containing pharmaceutical composition according to the present invention includes N terminus (aa 1-35)-deleted and C terminus (aa 271-352)-deleted DV ΔNC NS1 protein, which is depleted of cross-reactivity with endothelial cells and platelets, is able to shorten bleeding time and does not cause antibody dependent enhancement and autoimmunity.

In the dengue vaccine-containing pharmaceutical composition according to the present invention, the N and C termini-deleted nonstructural protein ΔNC NS1 preferably has the amino acid sequence of SEQ.ID.NO.1.

The dengue vaccine-containing pharmaceutical composition according to the present invention preferably is used to treat or prevent dengue hemorrhagic fever or dengue shock syndrome.

In the dengue vaccine-containing pharmaceutical composition according to the present invention, the amino acid sequence similarity or identity between the N and C termini-deleted nonstructural protein ΔNC NS 1 and the SEQ.ID.NO.1 preferably is 90% or more, and more preferably is 95% or more.

The present invention also provides a nucleotide molecule with a sequence that encodes N and C termini-deleted nonstructural protein ΔNC NS 1. Herein, the N and C termini-deleted nonstructural protein ΔNC NS1 contains DV NS1 from amino acids 36 to 270.

The N and C termini-deleted nonstructural protein Δ NC NS 1 encoded from the nucleotide molecule of the present invention is able to act as a dengue vaccine and shorten bleeding time, depleted of cross-reactivity with endothelial cells and platelets, and does not cause antibody dependent enhancement and autoimmunity.

The nucleotide molecule of the present invention preferably has the sequence of SEQ.ID.NO.2 in the sequence listing. Preferably, SEQ.ID.NO.2 is obtained from the nucleotide sequence of dengue virus, which is shown as SEQ.ID.NO.4 in the sequence listing.

The nucleotide molecule of the present invention preferably is used to produce a dengue vaccine.

### BRIEF DESCRIPTION OF THE DRAWINGS

FIG. 1 shows the results of antibody binding to platelet assay *in vitro* according to one preferred example of the present invention;
FIG. 2 shows the results of antibody binding to platelet assay on passively immunized mice according to one preferred example of the present invention;
FIG. 3 shows the results of bleeding time tests on actively immunized mice according to one preferred example of the present invention; and
FIG. 4 shows the results of antibody binding to endothelial cell assay *in vitro* according to one preferred example of the present invention.

### DETAILED DESCRIPTION OF THE PREFERRED EMBODIMENT

### [Mice]

C3H/HeN mice were obtained from The Jackson Laboratory and maintained on standard laboratory food and water in the Laboratory Animal Center of National Cheng Kung University Medical College. Their 8-wk-old progeny were used for the experiments. Housing, breeding, and experimental use of the animals were performed in strict accordance with the Experimental Animal Committee in National Cheng Kung University.

### [Platelet Preparation]

Human whole blood containing anticoagulant (29.9 mM sodium citrate, 113.8 mM glucose, 72.6 mM NaCl, and 2.9 mM citric acid (pH 6.4)) was centrifuged at 100xg for 20 min at room temperature to obtain platelet-rich plasma (PRP). The platelet-rich plasma was centrifuged at 1000xg for 10 min at room temperature and washed in EDTA/PBS buffer twice. The washed platelets were suspended in Tyrode's solution (137 mM NaCl, 20 mM HEPES, 3.3 mMNaH₂PO₄, 2.4 mM KCl, 1 mg/ml BSA, and 5.6 mM glucose (pH 7.4)) at a concentration of 10⁸ platelets/ml.

### [cDNA ]

Dengue type 2 (New Guinea C strain) virus grown in BHK21 cells was available from National Institute of Preventive Medicine, Department of Health, and cDNA were obtained. *Escherichia coli* BL-21(DE3) pLysS strain bearing the plasmid pRSET-DVNS1 was used to express recombinant proteins of DV NS1 and was available from Shiau's lab (Dr. Ai-Li Shiau) of Department of Microbiology and Immunology of National Cheng Kang University (Taiwan).

### [DV ΔNC NS1 from cDNA]

Primers P3758, P3759, P3760 and P3761 were used to amplify DV ΔNC NS1 cDNA from the pPRSET-DVNS1. Herein, PCR was performed as follows.
〈〈 Primer 〉〉
P3758 : 5' -AATTCCCAGAATCCCCTTCAAAACTG-3';
P3759:5' - CCCAGAATCCCCTTCAAAACTG-3;
P3760:5' -TCGAGTCATAAATGCCATGGTCC-3';
P3761:5'- GTCATAAATGCCATGGTCCTGCTAT-3'.

PCR primers, pPRSET-DVNS1 as a template and reaction enzymes (including DNA polymerase, T4 polynucleotide kinase and deoxymononucleotide) were placed into two centrifuge tubes, respectively, and uniformly mixed to perform a denaturation step (95 °C for 5 min) and a renaturation step (65 °C for 10 min). About 25% of PCR products were DV ΔNC NS1 cDNAs with cohesive ends containing *Eco*RI and *Xho*lI restriction sites. The ligation was accomplished by insertion of PCR products into a vector (pET28a).

### [Recombinant protein and Antibody preparation]

JEV NS 1, DV NS 1, and N terminus (aa 1-35)-deleted and C terminus (aa 271-352)-deleted dengue virus nonstructural protein 1 (DV ΔNC NS1) cDNA were cloned into the above-mentioned vector with histidine-tag. Plasmids were introduced into *Escherichia coli* BL21. The recombinant proteins were induced by 1 ┌ µM isopropyl B-D-1-thiogalactopyranoside (Calbiochem) and purified with Ni²⁺ columns. Subsequently, proteins were examined using 10% SDS-PAGE. Proteins from SDS-PAGE were excised and homogenized in adjuvant to immunize mice. Purified protein (25 µg) was emulsified in CFA for the first immunization, and 2 wk later in IFA for 2, 3 or 4 immunizations every week. Mouse sera were collected 3 days after the last immunization, and IgG was purified using protein G columns (Pharmacia Fine Chemicals).

### [Antibody binding to platelet assay]

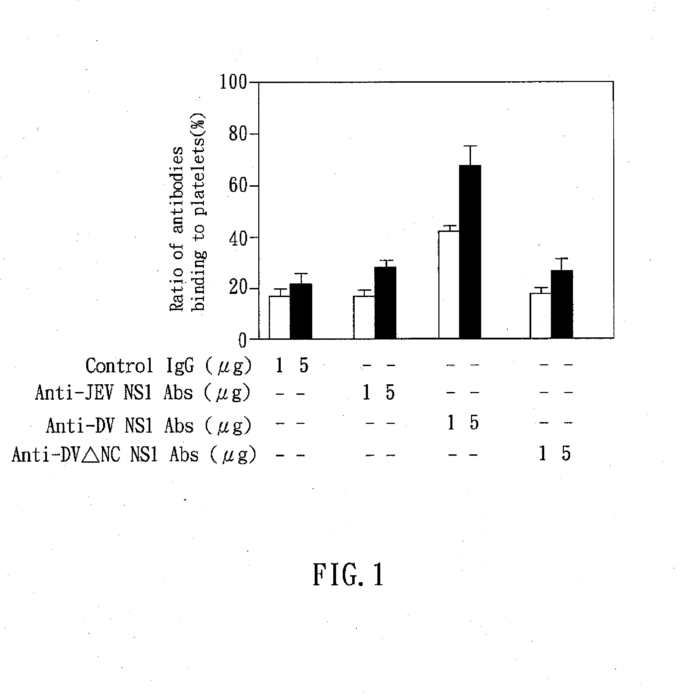

Washed platelets were fixed with 1% formaldehyde in PBS at room temperature for 10 min and then washed with PBS. Various doses of anti-full-length DV NS1, anti- DV ΔNC NS1 or anti-JEV NS1 were incubated with platelets for 30 min. After washing, platelets were incubated with FITC-conjugated anti-mouse IgG (Jackson ImmunoResearch Laboratories) for 30 min. Antibody binding to platelets was analyzed using flow cytometry, as shown in FIG. 1.

As shown in FIG. 1, the binding ability of anti-DV ΔNC NS1 antibody to human platelets was lower than that of anti-full-length DV NS 1 antibody, and similar to that of control IgG and anti-JEV NS1.

### [Antibody binding to platelet assay -Animal Model]

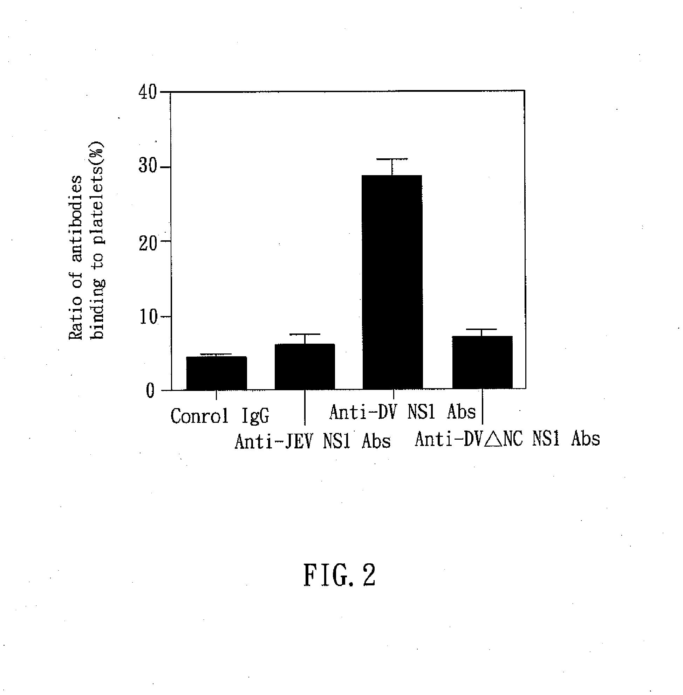

The antibody binding to platelet assay was performed on passively immunized mice with IgG (as a control), anti-full-length DV NS 1, anti-DV ΔNC NS1 or anti-JEV NS1 Abs. The results are shown in FIG. 2.

It can be found that the binding ability of anti-DV ΔNC NS 1 antibody to platelets was significantly lower than that of anti-full-length DV NS1 antibody, and similar to that of control IgG and anti-JEV NS1.

### [Bleeding time]

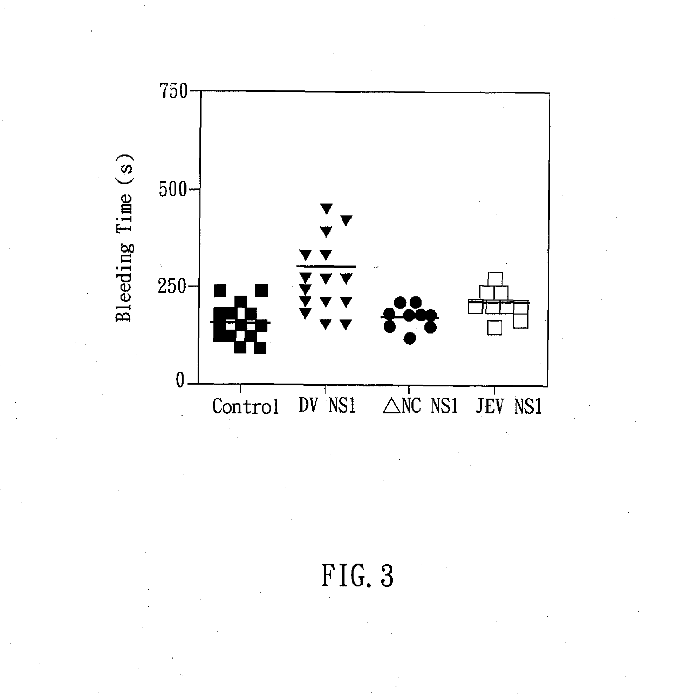

Actively immunized mice with full-length DV NS1, DV ΔNC NS1 and JEV NS 1 proteins and normal control mice with no above-mentioned proteins were tested on bleeding time. Bleeding time was performed by a 3-mm tail-tip transection. Blood droplets were collected on filter paper every 30 s for the first 3 min, and every 10 s thereafter. Bleeding time was recorded when the blood spot was smaller than 0.1 mm in diameter. The results are shown in FIG. 3.

As shown in FIG. 3, the bleeding time in N terminus (aa 1-35)-deleted and C terminus (aa 271-352)-deleted dengue virus nonstructural protein 1 (DV ΔNC NS1)-immunized mice was shorter than that in full-length DV NS1-immunized mice, and similar to that in normal control mice and JEV NS 1-immunized mice. The results show that the dengue vaccine including N terminus (aa 1-35)-deleted and C terminus (aa 271-352)-deleted dengue virus nonstructural protein 1 (DV ΔNC NS1) according to the present invention indeed has the effect for shortening bleeding time.

### [Antibody binding to endothelial cell assay]

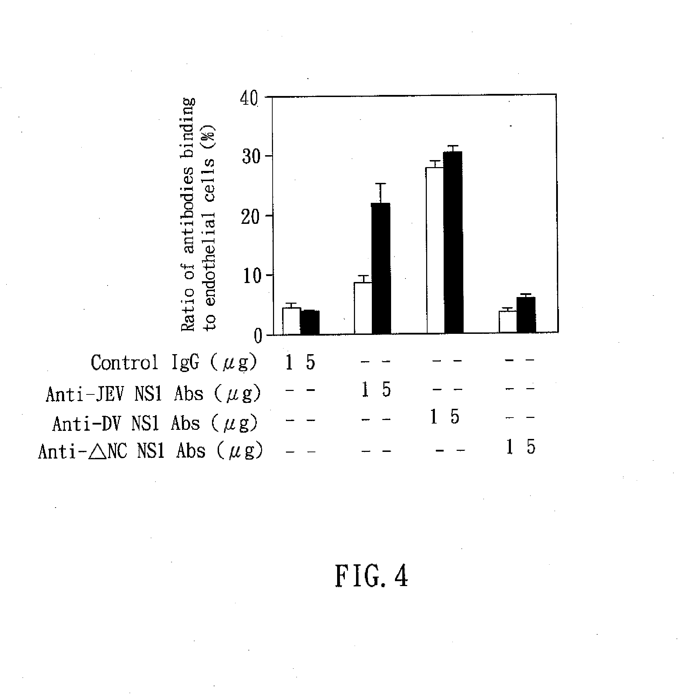

The human microvascular endothelial cell line (HMEC-1) was available from Center for Disease Control and Prevention, Atlanta. GA. The cells were cultured at 37°C and washed by PBS, followed by the addition of trypsin-EDTA, and reacted for several minutes. Then, the cells were re-suspended in a fresh serum-containing medium in an appropriate amount. After centrifugation, the cells were fixed with 1% formaldehyde in PBS for 10 min and then washed with PBS. Diluted antibodies in an appropriate dose were incubated with the cells for 1 hr. After washing with PBS for three times, the cells were incubated with FITC-conjugated secondary antibodies for 1 hr. The amount of antibody binding to endothelial cells was analyzed using flow cytometry, as shown in FIG. 4.

The results show that the binding ability of the anti-DV ΔNC NS 1 antibody according to the present invention to endothelial cells was significantly lower than that of anti-full-length DV NS1 antibody, and similar to that of IgG antibody (as a control) and anti-JEV NS 1 antibody. Therefore, it can be confirmed that the anti-DV ΔNC NS1 antibody according to the present invention does not cause autoimmunity.

Through sequence alignment analysis, the inventors of the present invention found that the N-terminal and C-terminal regions of DV NS 1 protein contain epitopes involved in cross-reactivity to target proteins. Therefore, the present invention evaluated and compared the influence of the full-length DV NS 1 protein and the N terminus (aa 1-35)-deleted and C terminus (aa 271-352)-deleted DV ΔNC NS1 on cross-reactivity and bleeding time. The experimental results showed that the binding ability of anti-DV ΔNC NS1 antibody to endothelial cells and platelets was lower than that of anti-full-length DV NS1 antibody. Through mouse models (animal models), it was found that the actively immunized mice with the full-length DV NS 1 protein showed prolonged bleeding time, while the phenomenon was not found in the immunized mice with the DV ΔNC NS 1 protein. The tests on passively immunized mice proved that the anti-DV ΔNC NS 1 antibody showed lower binding ability to platelets and thus had better effect for the reduction of bleeding time compared to the anti-full-length DV NS1 antibody.

In conclusion, the vaccine including N and C termini-deleted DV Δ NC NS1 protein according to the present invention is depleted of cross-reactivity with endothelial cells and platelets, shortens bleeding time, and can avoid autoimmunity. In addition, DV Δ NC NS1 protein of the present invention is derived from dengue virus nonstructural protein rather than dengue virus envelope protein, and thus can be used as an inventive and practical vaccine without antibody dependent enhancement.

Although the present invention has been explained in relation to its preferred embodiment, it is to be understood that many other possible modifications and variations can be made without departing from the spirit and scope of the invention as hereinafter claimed.

## Claims

1. A dengue vaccine, comprising:
N and C termini-deleted nonstructural protein ΔNC NS1 with a peptide fragment from amino acids 36 to 270, which is derived from dengue virus nonstructural protein 1 (DV NS1) with deletions of N-terminal region from amino acids 1 to 35 and C-terminal region from amino acids 271 to 352.

2. The dengue vaccine as claimed in claim 1, wherein the amino acid sequence similarity between the N and C termini-deleted nonstructural protein ΔNC NS1 and the SEQ.ID.NO.1 is 90% or more.

3. The dengue vaccine as claimed in claim 1 or 2, wherein the amino acid sequence similarity between the N and C termini-deleted nonstructural protein ΔNC NS1 and the SEQ.ID.NO.1 is 95% or more.

4. The dengue vaccine as claimed in any one of claims 1 to 3, wherein the N and C termini-deleted nonstructural protein ΔNC NS1 has the amino acid sequence of SEQ.ID.NO.1.

5. The dengue vaccine as claimed in any one of claims 1 to 4, wherein the N and C termini-deleted nonstructural protein ΔNC NS1 is derived from dengue virus protein.

6. The dengue vaccine as claimed in any one of claims 1 to 5 for use in the prevention or treatment of dengue hemorrhagic fever or dengue shock syndrome.

7. A dengue vaccine-containing pharmaceutical composition, wherein the dengue vaccine comprises:
N and C termini-deleted nonstructural protein ΔNC NS1 with a peptide fragment from amino acids 36 to 270, which is derived from dengue virus nonstructural protein 1 (DV NS1) with deletions of N-terminal region from amino acids 1 to 35 and C-terminal region from amino acids 271 to 352.

8. The dengue vaccine-containing pharmaceutical composition as claimed in claim 7, wherein the amino acid sequence similarity between the N and C termini-deleted nonstructural protein ΔNC NS1 and the SEQ.ID.NO.1 is 90% or more.

9. The dengue vaccine-containing pharmaceutical composition as claimed in claim 7 or 8, wherein the amino acid sequence similarity between the N and C termini-deleted nonstructural protein ΔNC NS1 and the SEQ.ID.NO.1 is 95% or more.

10. The dengue vaccine-containing pharmaceutical composition as claimed in any one of claims 7 to 9, wherein the N and C termini-deleted nonstructural protein Δ NC NS1 has the amino acid sequence of SEQ.ID.NO.1.

11. The dengue vaccine-containing pharmaceutical composition as claimed in any one of claims 7 to 10 for use in the prevention or treatment of dengue hemorrhagic fever or dengue shock syndrome.

12. A nucleotide molecule that encodes N and C termini-deleted nonstructural protein ΔNC NS1, wherein the N and C termini-deleted nonstructural protein ΔNC NS1 comprises dengue virus nonstructural protein 1 (DV NS 1) from amino acids 36 to 270.

13. The nucleotide molecule as claimed in claim 12, which is SEQ.ID.NO.2.

14. The nucleotide molecule as claimed in claim 12 or 13, which is used to produce a dengue vaccine.
